# EUROPEAN PATENT APPLICATION

(11) **EP 4 411 514 A1**
(43) Date of publication of application: **07.08.2024**
(21) Application number: 22876525.1
(22) Date of filing: 30.09.2022
(51) Int. Cl.: G06F 3/01, A23L 5/00, G06K 19/06, H04N 7/18

(54) **FOOD PRODUCT, SENSATION PRESENTATION SYSTEM, AND SENSATION PRESENTATION METHOD**

(30) Priority: 01.10.2021 JP 2021162720
(71) Applicant: DENTSU INC., Tokyo 105-7001 (JP)
(72) Inventor: SAKAKI Ryosuke, Tokyo 105-7001 (JP)
(74) Representative: Bardehle Pagenberg Partnerschaft mbB Patentanwälte Rechtsanwälte
(86) International application number: PCT/JP2022/036646
(87) International publication number: WO 2023/054662

(57) **Abstract**

A new sensory presentation system and sensory presentation method using a food, and a food that can be used therefor are provided.

A food having a readable marker associated with a visual, auditory, tactile, or olfactory effect is provided.

## Description

### Technical Field

The present invention relates to a food, a sensory presentation system, and a sensory presentation method.

### Background Art

Patent Literature 1 discloses a display control device that displays an image in which an appearance of a food or beverage is changed.

### Citation List

### Patent Literature

Patent Literature 1: WO 2014-97706 A

### Summary of Invention

### Technical Problem

An object of the present invention is to provide a new sensory presentation system and sensory presentation method using a food, and a food that can be used therefor.

### Solution to Problem

According to one embodiment of the invention, provided is a food comprising a readable marker associated with a visual, auditory, tactile, or olfactory effect.

The food may have a polyhedral shape and has the marker on at least one surface thereof.

The marker may be edible.

According to another embodiment of the invention, provided is a sensory presentation system comprising: the above food; a reading device that reads the marker; and a presentation device that presents an effect associated with the marker in response to reading of the marker.

The reading means may be a camera, the presentation device may be a display, and the effect may be a visual effect.

The sensory presentation system may comprises an HMD having the camera and the display.

When at least a part of the marker in the food is eaten, the effect presented by the presentation device may disappear.

The sensory presentation system may comprises: a database that stores the marker and the effect in association with each other; and a controller that refers to the database and controls the presentation device so that the effect associated with the read marker is presented.

The presentation device may include at least one of: a display capable of presenting a visual effect; a speaker capable of presenting an auditory effect; a blower capable of presenting a tactile effect; and a scent generator capable of presenting an olfactory effect.

According to another embodiment of the invention, provided is a sensory presentation method comprising: a step of reading, by a reading device, a readable marker associated with a visual, auditory, tactile, or olfactory effect that the food has; and a step of presenting, by a presentation device, an effect associated with the marker in response to reading of the marker.

### Brief Description of Drawings

Fig. 1 is a schematic perspective view of a food 10 used in a first embodiment.
Fig. 2 is a diagram for describing an outline of the first embodiment.
Fig. 3 is a block diagram illustrating a schematic configuration of a sensory presentation system according to the first embodiment.
Fig. 4 is a diagram illustrating information stored in a database 24.
Fig. 5 is a flowchart illustrating an example of a processing operation of the sensory presentation system according to the first embodiment.
Fig. 6A is a view illustrating a state in which a marker 12 is not visible.
Fig. 6B is a view illustrating a screen displayed on a display 21 in the state of Fig. 6A.
Fig. 7A is a view illustrating a state in which the marker 12 is visible.
Fig. 7B is a view illustrating a screen displayed on the display 21 in the state of Fig. 7A.
Fig. 8A is a view illustrating a state in which the marker 12 is no longer visible.
Fig. 8B is a view illustrating a screen displayed on the display 21 in the state of Fig. 8A.
Fig. 9 is a diagram illustrating an example of a sensory presentation system according to a second embodiment.
Fig. 10 is a diagram illustrating information stored in a database 24.

### Description of Embodiments

Hereinafter, an embodiment according to the present invention will be specifically described with reference to the drawings.

### (First Embodiment)

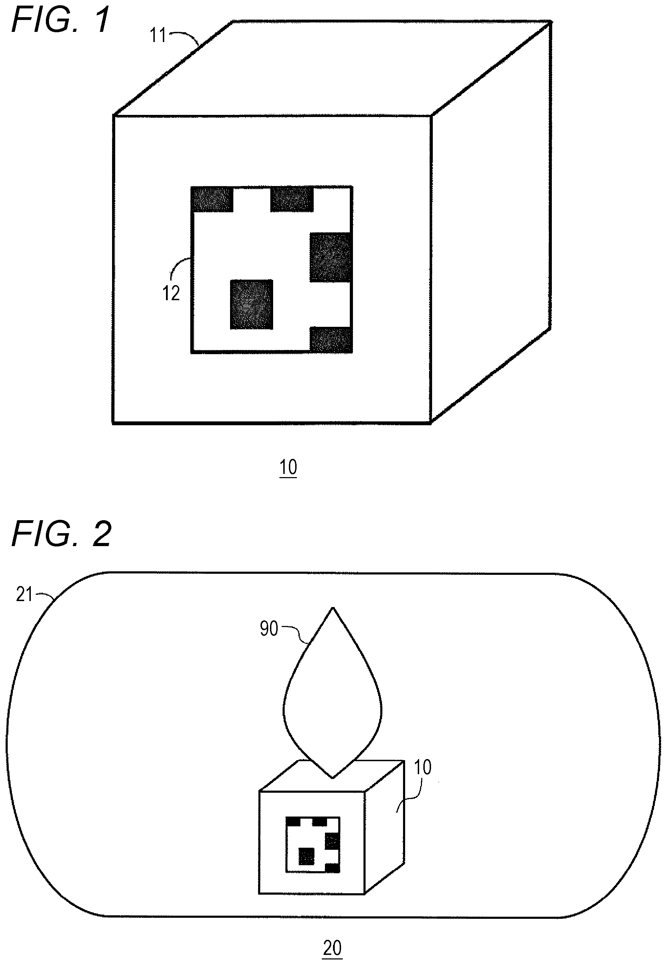

Fig. 1 is a schematic perspective view of a food 10 used in a first embodiment. The food 10 includes a food main body 11 and a marker 12 attached to the food main body 11.

The food main body 11 is not particularly limited, but is desirably a polyhedral solid, for example, a substantially rectangular parallelepiped chocolate. The food main body 11 may be placed in a container (desirably, an edible container) having a predetermined shape.

The marker 12 is desirably formed of edible food such as red food coloring, and is provided on a plane of the food main body 11. The marker 12 may be a bar code, a QR code (registered trademark), an AR marker, or the like. A visual effect is associated with the marker 12. In detail, the marker 12 is readable by a reading device described later, and a visual effect corresponding to the marker 12 is presented to the user by being read.

As an example, the reading device is a head mount display (HMD) 20. Then, as illustrated in Fig. 2, when the food 10 is read by the HMD 20, a flame 90 appears on the food 10 on a display 21 of the HMD 20 as a visual effect. Details will be described below.

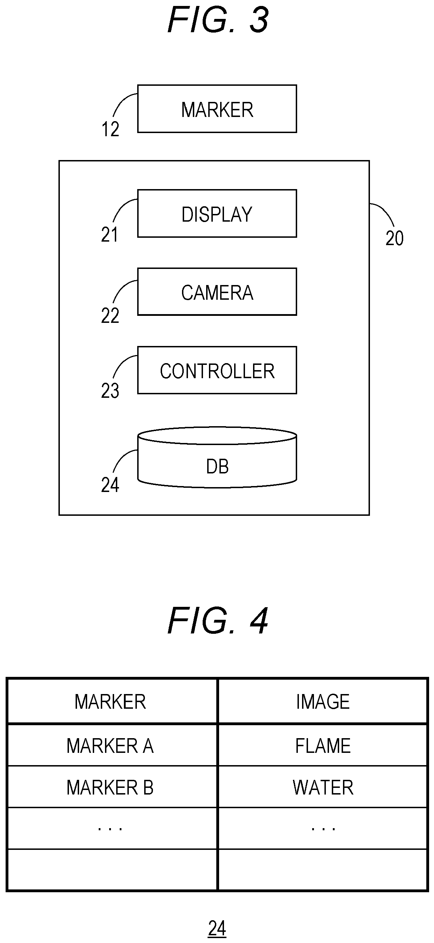

Fig. 3 is a block diagram illustrating a schematic configuration of the sensory presentation system according to the first embodiment. The sensory presentation system includes the food 10 having the marker 12 described above and the HMD 20.

The HMD 20 includes a display 21, a camera 22, a controller 23, and a database 24. The display 21 is located in front of both eyes of the user when the user wears the HMD 20 on the head. The camera 22 captures an image of a front of the HMD 20. The controller 23 controls a video displayed on the display 21 and processes an image captured by the camera 22. In the database 24, a relationship between the marker 12 and a visual effect (for example, an image to be displayed) is stored in advance (see Fig. 4).

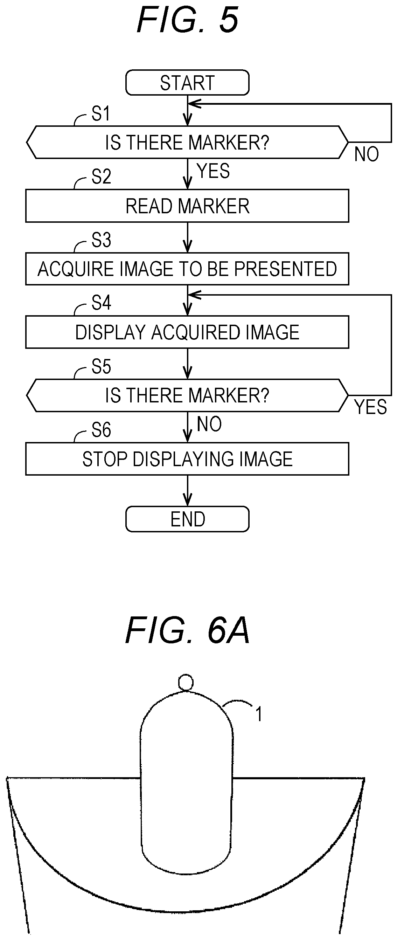

Fig. 5 is a flowchart illustrating an example of a processing operation of the sensory presentation system according to the first embodiment.

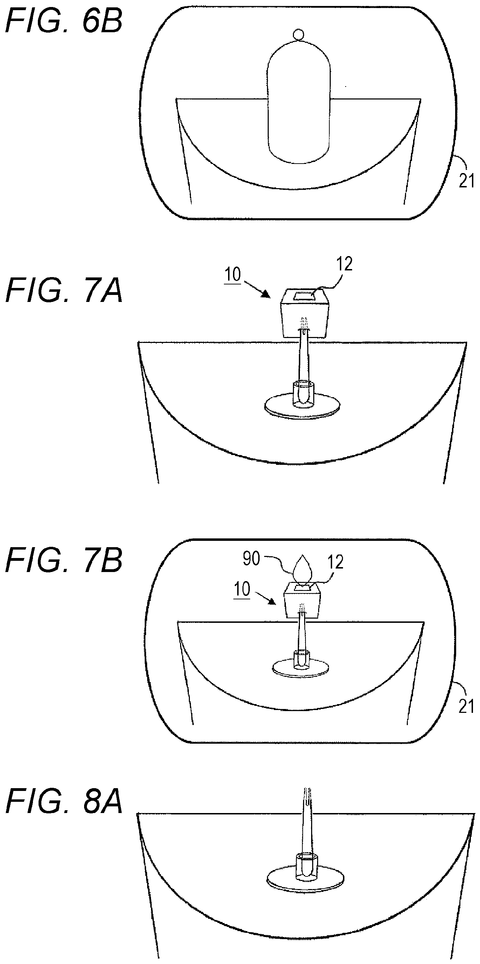

For example, a meal including the food 10 is provided to the user wearing the HMD 20, but the food 10 is initially covered by the cover 1 and is not visible to the user (Fig. 6A). Of course, the marker 12 attached to the food 10 cannot be read by the camera 22 of the HMD 20 (NO in step S1 in Fig. 5). At this time, only a video captured by the camera 22 is displayed on the display 21 of the HMD 20 (Fig. 6B).

When the user removes the cover 1, the food 10 appears (Fig. 7A). Thus, the marker 12 of the food 10 enters an imaging range of the camera 22 (YES in step S1 in Fig. 5), and the camera 22 reads the marker 12 (step S2). The controller 23 acquires an image corresponding to the marker 12 read by the camera 22 from the database 24 (step S3).

Then, the controller 23 causes the display 21 to display the acquired image (step S4). In other words, in response to the marker 12 being read by the camera 22, the display 21 displays an image associated with the marker 12.

As an example, as illustrated in Fig. 7B, the flame 90 is superimposed and displayed on the video captured by the camera 22 on the display 21. As the position where the flame 90 is to be displayed, for example, a positional relationship with the marker 12 may be stored in the database 24.

While the marker 12 of the food 10 is within the imaging range of the camera 22 (YES in step S5), the controller 23 recognizes the fact and continues to display the image on the display 21 (step S4). That is, while the marker 12 of the food 10 is in the imaging range of the camera 22, the display 21 continues to display the image.

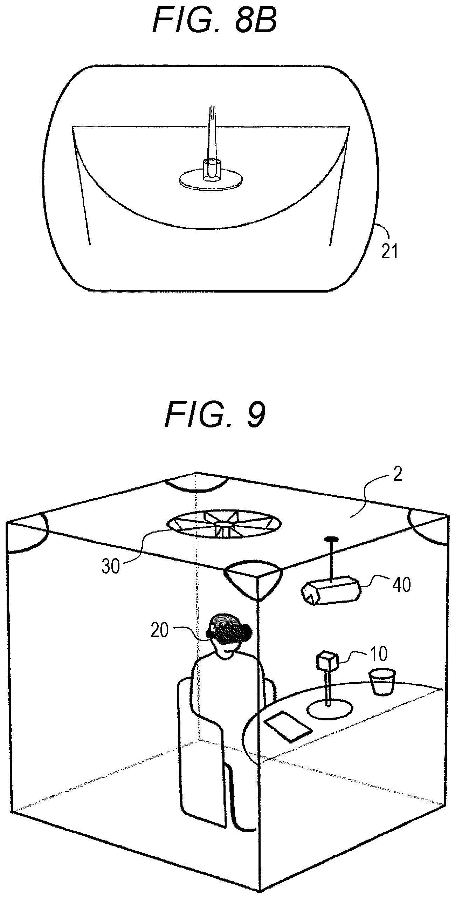

When, for example, a part or the entirety of the marker 12 on the food 10 is eaten and thus the marker 12 on the food 10 is out of the imaging range of the camera 22 (NO in step S5, Fig. 8A), the controller 23 cannot read the marker 12, and stops displaying the image that has been displayed on the display 21 (step S6). That is, when the marker 12 of the food 10 is out of the imaging range of the camera 22, the display 21 stops displaying the image (Fig. 8B).

Note that the display 21 may be a transmissive display, and the display/non-display of the flame 90 may be controlled without displaying the video captured by the camera 22 on the display 21.

As described above, in the first embodiment, the visual effect associated with the marker 12 can be presented to the user by providing the marker 12 on the food 10.

### (Second Embodiment)

A second embodiment described below is an evolution of the first embodiment, and enables presentation of not only visual effects but also an auditory, tactile, or olfactory effect. Hereinafter, differences from the first embodiment will be mainly described.

Fig. 9 is a diagram illustrating an example of a sensory presentation system according to the second embodiment. The sensory presentation system includes the food 10, an HMD 20, a blower 30, and a scent generator 40, which may be disposed in a stereophonic sound space 2.

As described in the first embodiment, the food 10 includes the marker 12. The marker 12 is not limited to a visual effect, and may be associated with an auditory, tactile, and/or olfactory effect. The marker 12 is readable by the camera 22 of the HMD 20, and the effect corresponding to the marker 12 is presented to the user by being read.

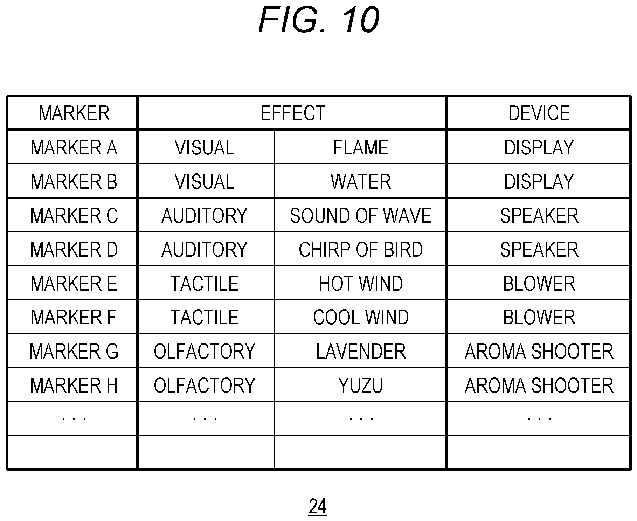

Although the HMD 20 is as described in the first embodiment, the relationship between the marker 12 and the presented effects is stored in advance in the database 24 (Fig. 3), and the effects are not limited to visual effects but may include an auditory, tactile, and/or olfactory effect (see Fig. 10).

By displaying various images on the display 21 of the HMD 20, a desired visual effect can be presented to the user. In addition, the HMD 20 may include a speaker (not illustrated), which can present an auditory effect to the user.

The blower 30 is installed, for example, on a ceiling in the stereophonic sound space 2 and blows air toward the user. By appropriately adjusting the strength, direction, temperature, and the like of the wind, a desired tactile effect can be presented to the user. The blower 30 is, for example, a fan or an air conditioner.

The scent generator 40 is installed, for example, on the ceiling in the stereophonic sound space 2 and injects a scent toward the user. A desired olfactory effect can be presented to the user by appropriately adjusting the type and strength of the scent (aromatic oil). The scent generator 40 is, for example, an aroma shooter.

Note that the display 21 and the speaker of the HMD 20, the blower 30, and the scent generator 40 described above are merely examples of devices (presentation devices) that present a predetermined effect to the user. Some of these may be omitted, or a visual, auditory, tactile, or olfactory effect may be presented to the user by another device (presentation device).

The processing operation of the sensory presentation system of Fig. 9 is substantially similar to that of the first embodiment except that the presented effect is not limited to a visual effect. That is, the controller 23 controls the presentation device according to the read marker 12, whereby the effect associated with the marker 12 is presented to the user.

As described above, it is possible to enjoy a meal in what is called a cyberspace.

Based on the above description, a person skilled in the art may be able to conceive additional effects and various modifications of the present invention, but aspects of the present invention are not limited to the individual embodiments described above. Various additions, modifications, and partial deletions can be made without departing from the conceptual idea and spirit of the present invention derived from the contents defined in the claims and equivalents thereof.

For example, what is described herein as a single device (including what is depicted in the drawings as a single device) may be implemented by a plurality of devices. Conversely, what is described herein as a plurality of devices (including what is depicted in the drawings as a plurality of devices) may be implemented by one device. Alternatively, some or all of means or functions assumed to be included in a certain device (for example, a server) may be included in another device (for example, a user terminal).

In addition, not all the matters described in the present specification are essential requirements. In particular, matters described in the present specification and not described in the claims can be regarded as any additional matters.

It should be noted that the applicant of the present invention is merely aware of the invention disclosed in the literature in the section of "Citation List" in the present description, and the present invention is not necessarily intended to solve the problem in the invention disclosed in the literature. The problem to be solved by the present invention should be recognized in consideration of the entire specification. For example, in the present specification, in a case where there is a description that a predetermined effect is exhibited by a specific configuration, it can be said that the problem of reversing the predetermined effect is solved. However, such a specific configuration is not necessarily an essential requirement.

### Reference Signs List

- 10: Food
- 11: Food main body
- 12: Marker
- 20: HMD
- 21: Display
- 22: Camera
- 23: Controller
- 24: DB
- 30: Blower
- 40: Scent generator

## Claims

1. A food comprising a readable marker associated with a visual, auditory, tactile, or olfactory effect.

2. The food according to claim 1, wherein the food has a polyhedral shape and has the marker on at least one surface thereof.

3. The food according to claim 1 or 2, wherein the marker is edible.

4. A sensory presentation system comprising:
the food according to any one of claims 1 to 3;
a reading device that reads the marker; and
a presentation device that presents an effect associated with the marker in response to reading of the marker.

5. The sensory presentation system according to claim 4, wherein
the reading means is a camera,
the presentation device is a display, and
the effect is a visual effect.

6. The sensory presentation system according to claim 5, comprising an HMD having the camera and the display.

7. The sensory presentation system according to any one of claims 4 to 6, wherein when at least a part of the marker in the food is eaten, the effect presented by the presentation device disappears.

8. The sensory presentation system according to any one of claims 4 to 7, comprising:
a database that stores the marker and the effect in association with each other; and
a controller that refers to the database and controls the presentation device so that the effect associated with the read marker is presented.

9. The sensory presentation system according to claim 4, wherein
the presentation device includes at least one of:
a display capable of presenting a visual effect;
a speaker capable of presenting an auditory effect;
a blower capable of presenting a tactile effect; and
a scent generator capable of presenting an olfactory effect.

10. A sensory presentation method comprising:
a step of reading, by a reading device, a readable marker associated with a visual, auditory, tactile, or olfactory effect that the food has; and
a step of presenting, by a presentation device, an effect associated with the marker in response to reading of the marker.
